# EUROPÄISCHE PATENTSCHRIFT

(11) **EP 0 671 467 B1**
(45) Veröffentlichungstag und Bekanntmachung des Hinweises auf die Patenterteilung: **09.08.2000**
(21) Anmeldenummer: 95101871.2
(22) Anmeldetag: 11.02.1995
(51) Int. Cl.: C12M 1/22

(54) **Positionierbare Petrischale**
Ajustable petri dish
Disque de petri ajustable

(30) Priorität: 02.03.1994 DE 4406725
(43) Veröffentlichungstag der Anmeldung: 13.09.1995
(73) Patentinhaber: HEIPHA GmbH, D-69115 Heidelberg (DE)
(72) Erfinder: Müller, Rolf, Dr., D-69221 Dossenheim (DE)
(74) Vertreter: Naumann, Ulrich, Dr.-Ing.

(56) Entgegenhaltungen:
- DE-B- 1 210 129
- FR-A- 424 534
- FR-A- 2 157 050
- FR-A- 2 300 012
- US-A- 3 006 461
- PATENT ABSTRACTS OF JAPAN vol. 012, no. 496 (C-555), 23.Dezember 1988 & JP 63 207377 A (ERUMETSUKUSU:KK), 26.August 1988,

## Beschreibung

Die Erfindung betrifft ein Behältnis zur Aufnahme von festen und/oder flüssigen Medien, insbesondere von Nährlösungen oder Nährböden für Bakterienkulturen, mit einem Boden, einer von dem Boden abragenden Seitenwandung und mindestens einer das Behältnis in getrennte Bereiche unterteilenden Trennwand.

Behältnisse der in Rede stehenden Art sind seit langem aus der Praxis bekannt und existieren in den unterschiedlichsten Ausführungsformen und -größen. Solche Behältnisse finden ihre Verwendung bspw. in der pharmazeutischen, chemischen oder biotechnologischen Industrie und dienen Herstellern von Fertignährlösungen bzw. Nährböden für Bakterienkulturen zur Aufnahme und dem Transport dieser Substanzen. Das Ein- bzw. Abfüllen von ggf. mehreren unterschiedlichen Fertignährlösungen bzw. Nährböden in die getrennten Bereiche einzelner Behältnisse erfolgt dabei üblicherweise mittels automatischer Abfüllmaschinen.

DE-A-1 210 129 offenbart eine abdeckbare Petri-Schale zum Züchten anaerober Kulturen, die einen Boden, eine vom Boden abragende Seitenwandung und zwei unterteilende Trennwände, die die Schale in vier getrennte Bereiche teilt, aufweist. Das Problem der automatischen Abfüllung von verschiedenen Bereichen wird in diesem Dokument nicht erwähnt.

JP-A-63 207 377 offenbart ein Gerät zur automatischen Fraktionierung und Pipettierung von Medien auf verschiedene getrennte Bereiche von Petri-Schalen. Hier werden die Einfüllstutzen der Abfüllmaschine positioniert.

Für einen reibungslosen Abfüllvorgang müssen die Behältnisse exakt unter die Einfüllstutzen einer Abfüllmaschine positioniert werden. Die erforderliche exakte Positionierung eines Behältnisses wird bislang mit Positioniersystemen durchgeführt, die mittels lichtoptischer Detektion der Position der Trennwand des Behältnisses arbeiten. Bei diesen Systemen ist jedoch problematisch, daß die Winkelposition der Trennwand relativ zu einer im wesentlichen senkrecht durch den Boden verlaufenden Drehachse nicht eindeutig bestimmt werden kann. Weist das Behältnis als Boden bspw. eine ebene Kreisfläche und als Trennwand eine das Behältnis in exakt zwei gleich große Bereiche teilende senkrechte Trennwand auf, können die bekannten Positioniersysteme keine eindeutige Positionierung des Behältnisses vornehmen, da in diesem Falle nämlich genau zwei um 180° gedrehte, durch das Positioniersystem nicht unterscheidbare Positionen der Trennwand bzw. des Behältnisses eingestellt sein können. Folglich wird eine eindeutige Zuordnung unterschiedlicher Nährlösungen bzw. Nährböden bei der Abfüllung zu vor der Abfüllung vorgegebenen Bereichen verhindert.

Der vorliegenden Erfindung liegt daher die Aufgabe zugrunde, ein Behältnis der eingangs genannten Art derart auszugestalten und weiterzubilden, daß eine eindeutige Winkelpositionierung des Behältnisses relativ zu einer im wesentlichen senkrecht durch den Boden verlaufenden Drehachse ermöglicht ist.

Erfindungsgemäß wird die voranstehende Aufgabe durch die Merkmale des Patentanspruches 1 gelöst. Danach ist das in Rede stehende Behältnis derart ausgebildet, daß an einer vorgegebenen Stelle des Behältnisses ein Anschlag zur Winkelpositionierung des Behältnisses relativ zu einer im wesentlichen senkrecht durch den Boden verlaufenden Drehachse angeordnet ist.

In erfindungsgemäßer Weise ist erkannt worden, daß die Anordnung eines Anschlags an einer vorgegebenen Stelle des Behältnisses die obengenannte Aufgabe auf überraschend einfache Weise löst. Das erfindungsgemäße Behältnis kann bspw. von einer Greifeinrichtung aus einem Regal unter die Einfüllstutzen einer Abfüllmaschine angeordnet werden und von derselben Greifeinrichtung oder auch einer anderen geeigneten Dreheinrichtung so lange um eine im wesentlichen senkrecht durch den Boden verlaufende Drehachse gedreht werden, bis der Anschlag an einem geeigneten Konterstück anliegt. Nach einer solchen Positionierung befindet sich das Behältnis in einer vorgegebenen und eindeutigen, wenn auch vorher beliebig gewählten Position.

Mit der erfindungsgemäßen Ausbildung des Behältnisses ist folglich ein Behältnis realisiert, das eine eindeutige Winkelpositionierung des Behältnisses relativ zu einer im wesentlichen senkrecht durch den Boden verlaufenden Drehachse ermöglicht.

Im Hinblick auf eine möglichst einfache Ausgestaltung des Abfüllsystems könnte das mit dem Anschlag des Behältnisses in Eingriff zu bringende Konterstück in Form eines Stiftes aus dem Untergrund herausfahrbar sein, auf welchem das Behältnis unter den Einfüllstutzen angeordnet ist. Hierfür wäre es von Vorteil, wenn der Anschlag auf der Unterseite des Bodens angeordnet ist und ggf. von der Unterseite des Bodens abragt.

Damit trotz des Vorhandenseins eines Anschlags an der Unterseite des Bodens eine stabile und kippfreie Auflage des Behältnisses auf einem Untergrund ermöglicht ist, könnte der Boden ein nach unten abragendes Auflageelement zur Auflage auf einem Untergrund aufweisen. In diesem Falle wäre es weiter vorteilhaft, wenn das Auflageelement mindestens so weit wie der Anschlag vom Boden abragt.

Zur Gewährleistung einer besonders stabilen Auflage und einer gleichzeitig harmonischen und homogenen Form des Behältnisses wäre eine Ausbildung des Auflageelements als zumindest teilweise entlang dem Bodenumfang verlaufender Bodenrand denkbar.

Hinsichtlich eines günstigen Verhältnisses zwischen Behältnisvolumen und Umfangsfläche wäre eine Ausbildung des Bodens als polygone Fläche oder besser noch als ebene Kreisfläche von Vorteil. Zur Erhöhung der Handlichkeit des Behältnisses könnte die Seitenwandung dann im wesentlichen senkrecht vom Boden abragen.

Zum Erreichen einer sauberen Trennung der Bereiche ist es vorteilhaft, wenn die Trennwand senkrecht zum Boden angeordnet ist.

Zur Ausbildung der getrennten Bereiche als gleichwertige und damit in gleicher Weise nutzbarer Bereiche könnte die Trennwand geradlinig durch die Mitte des Behältnisses verlaufen. Damit wäre das Behältnis in zwei vorzugsweise gleich große Bereiche aufgeteilt. In gleicher Weise wäre jedoch auch denkbar, daß zur Aufteilung des Behältnisses in mehrere vorzugsweise gleich große Bereiche mehrere Trennwände vorgesehen sind. Hierzu könnten die Trennwände weiterhin sternförmig mit einem gemeinsamen Schnittpunkt in der Mitte des Behältnisses angeordnet sein.

Im Hinblick auf eine harmonische und möglichst unauffällige Integration des Anschlags in den Aufbau des Behältnisses könnte der Anschlag stegförmig als quasi Verlängerung einer Trennwand nach unten ausgebildet sein und bei einem als ebene Kreisfläche ausgebildeten Boden eine geringere Länge als der Radius der Kreisfläche des Bodens aufweisen.

Eine besonders stabile und einfach zu erzeugende Ausgestaltung des Behältnisses wäre durch die Ausbildung des Bodens, der Seitenwandung, der Trennwand bzw. der Trennwände, des Anschlags und ggf. des Auflageelements als integrale Bestandteile des Behältnisses erreicht.

Beim Handhaben aufgenommener Medien ist es von großem Vorteil, wenn das Behältnis transparent ist. Hierfür wäre eine Ausbildung des Behältnisses aus Glas oder aus Kunststoff denkbar. Diese Materialien sind desweiteren bei Verwendung des Behältnisses unter sterilen Bedingungen vorteilhaft, da sie für eine Wiederverwendung einfach reinig- bzw. sterilisierbar sind. Ein aus Kunststoff ausgebildetes Behältnis könnte dabei spritzgußtechnisch hergestellt sein.

Zur Gewährleistung einer bequemen Unterscheidung der einzelnen Bereiche könnten am Boden den einzelnen Bereichen zugeordnete, unterschiedliche Kennzeichnungen vorgesehen sein. Diese Kennzeichnungen könnten eingegossen oder eingeprägt sein. Hinsichtlich einer einfachen Erkennung könnten die Kennzeichnungen als alphanumerische Zeichen, als arabische oder auch als römische Ziffern, ausgebildet sein.

Im Hinblick auf eine problemlose Positionierbarkeit des Behältnisses ist es von Vorteil, wenn die Seitenwandung derart stabil ausgebildet ist, daß sie von einer Einrichtung zum Drehen des Behältnisses von oben greifbar ist.

In besonders vorteilhafter Weise könnte ein Deckel zum Verschließen des Behältnisses vorgesehen sein. Hierbei könnte die Seitenwandung von dem Deckel übergreifbar sein.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im allgemeinen bevorzugte Ausgestaltungen und Weiterbildungen der Lehre erläutert. In der Zeichnung zeigen
- Fig. 1: in einer Draufsicht ein Ausführungsbeispiel eines erfindungsgemäßen Behältnisses und
- Fig. 2: in einer Seitenansicht das Ausführungsbeispiel eines erfindungsgemäßen Behältnisses aus Fig. 1.

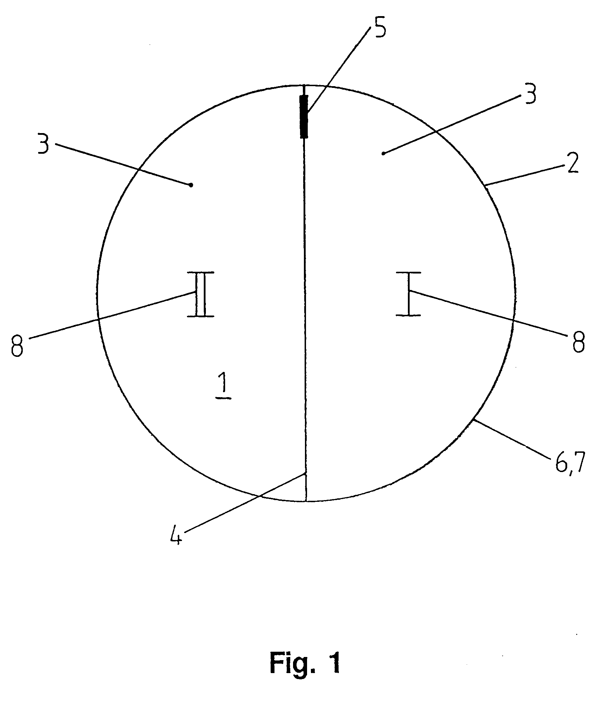

Fig. 1 zeigt in einer Draufsicht ein Ausführungsbeispiel eines erfindungsgemäßen Behältnisses zur Aufnahme von festen und/oder flüssigen Medien. Das Behältnis weist einen Boden 1, eine von dem Boden 1 abragende Seitenwandung 2 und eine das Behältnis in getrennte Bereiche 3 unterteilende Trennwand 4 auf. Zur Winkelpositionierung des Behältnisses relativ zu einer im wesentlichen senkrecht durch den Boden 1 verlaufenden Drehachse ist an einer vorgegebenen Stelle des Behältnisses ein Anschlag 5 angeordnet. Dieser Anschlag 5 ist im Randbereich des Behältnisses auf der Unterseite des Bodens 1 angeordnet, wobei er von der Unterseite des Bodens 1 abragt. Im Prinzip kann der Anschlag 5 an einer beliebigen vorgegebenen Stelle des Behältnisses ausgebildet sein. Zur Gewährleistung einer sicheren Positionierung des Behältnisses muß jedoch sichergestellt sein, daß der Anschlag 5 exzentrisch zu der gewählten Drehachse angeordnet ist.

Demnach wäre auch eine exzentrische Anordnung des Anschlags 5 an der Seitenwandung 2, der Trennwand 4 oder innerhalb eines der Bereiche 3 denkbar.

Das in Fig. 1 dargestellte Behältnis weist ein als Bodenrand 7 ausgebildetes Auflageelement 6 auf, um ein stabiles und kippfreies Auflegen des Behältnisses auf einem Untergrund trotz des nach unten abragenden Anschlags 5 zu ermöglichen. Der Bodenrand 7 verläuft dabei entlang dem Bodenumfang und ragt mindestens so weit wie der Anschlag 5 vom Boden 1 ab.

Der Boden 1 ist bei diesem Ausführungsbeispiel als ebene Kreisfläche ausgebildet. Eine Ausbildung des Bodens 1 als polygone Fläche wäre jedoch auch denkbar. Die Seitenwandung 2 und die Trennwand 4 sind senkrecht zum Boden 1 angeordnet bzw. ragen von diesem ab. Die Trennwand 4 verläuft dabei geradlinig durch die Mitte des Behältnisses und teilt damit das Behältnis in zwei gleich große Bereiche 3. Zur Bereitstellung weiterer gleich großer Bereiche 3 könnten mehrere Trennwände vorgesehen sein. Eine sternförmige Anordnung geradliniger Trennwände 4 mit einem gemeinsamen Schnittpunkt in der Mitte des Behältnisses würde eine solche Aufteilung in gleich große Bereiche 3 realisieren.

Zur Unterscheidung der einzelnen Bereiche 3 sind am Boden 1 den einzelnen Bereichen 3 zugeordnete, unterschiedliche Kennzeichnungen 8 angeordnet. Die Kennzeichnungen 8 können in dem Boden 1 eingegossen oder eingeprägt sein und sind als römische Ziffern ausgebildet. Eine Ausbildung der Kennzeichnungen 8 als alphanumerische Zeichen ist jedoch auch möglich.

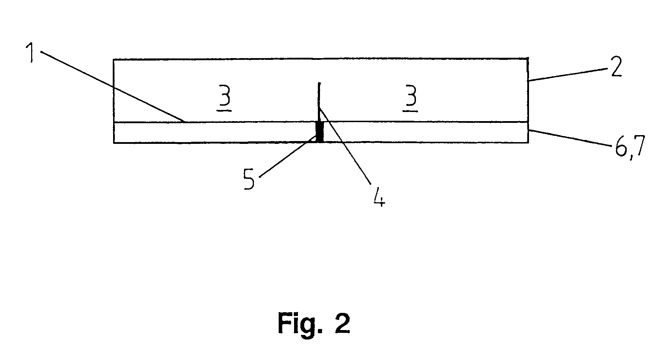

Fig. 2 zeigt eine Seitenansicht des Ausführungsbeispiels eines erfindungsgemäßen Behältnisses aus Fig. 1. Die Seitenwandung 2 ragt senkrecht vom Boden 1 ab und bildet mit dem Boden 1 und der senkrecht zum Boden 1 angeordneten Trennwand 4 die getrennten Bereiche 3. In dieser Seitenansicht ist besonders gut zu erkennen, daß das Ausführungsbeispiel die flache Schalenform einer sogenannten Petri-Schale aufweist und daß der Anschlag 5 auf der Unterseite des Bodens 1 angeordnet ist und von dort nach unten abragt. Das als entlang dem Bodenumfang verlaufender Bodenrand 7 ausgebildete Auflageelement 6 zur Auflage auf einem Untergrund ragt von dem Boden 1 gleich weit wie der Anschlag 5 ab. Dadurch ist eine stabile Auflage des Behältnisses auf einem Untergrund trotz eines nach unten abragenden Anschlags 5 gewährleistet.

Der Anschlag 5 ist stegförmig als quasi Verlängerung einer Trennwand 4 nach unten ausgebildet. Wie in Fig. 1 zu sehen ist, weist der stegförmige Anschlag 5 eine geringere Länge als der Radius der ebenen Kreisfläche des Bodens 1 auf.

Das in den Fig. 1 und 2 dargestellte Ausführungsbeispiel eines erfindungsgemäßen Behältnisses weist den Boden 1, die Seitenwandung 2, die Trennwand 4, den Anschlag 5 und das Auflageelement 6 bzw. den Bodenrand 7 als integrale Bestandteile auf. Als Herstellungsmaterial ist Glas oder Kunststoff vorgesehen, wobei ein aus Kunststoff hergestelltes Behältnis spritzgußtechnisch produziert sein kann.

Zum Verschließen des Behältnisses kann ein Deckel vorgesehen sein, der bspw. die Seitenwandung 2 übergreift.

Abschließend sei hervorgehoben, daß das zuvor rein willkürlich gewählte Ausführungsbeispiel lediglich zur Erörterung der erfindungsgemäßen Lehre dient, diese jedoch nicht auf dieses Ausführungsbeispiel einschränkt.

## Patentansprüche

1. Behältnis zur Aufnahme von festen und/oder flüssigen Medien, insbesondere von Nährlösungen oder Nährböden für Bakterienkulturen, mit einem Boden (1), einer von dem Boden (1) abragenden Seitenwandung (2) und mindestens einer das Behältnis in getrennte Bereiche (3) unterteilenden Trennwand (4),
**dadurch gekennzeichnet**, daß an einer vorgegebenen Stelle des Behältnisses ein Anschlag (5) zur Winkelpositionierung des Behältnisses relativ zu einer im wesentlichen senkrecht durch den Boden (1) verlaufenden Drehachse angeordnet ist.

2. Behältnis nach Anspruch 1, dadurch gekennzeichnet, daß der Anschlag (5) auf der Unterseite des Bodens (1) angeordnet ist und von der Unterseite des Bodens (1) abragt.

3. Behältnis nach Anspruch 2, dadurch gekennzeichnet, daß der Boden (1) ein nach unten abragendes Auflageelement (6) zur Auflage auf einem Untergrund aufweist, daß das Auflageelement (6) mindestens so weit wie der Anschlag (5) vom Boden (1) abragt und ggf. als zumindest teilweise entlang dem Bodenumfang verlaufender Bodenrand (7) ausgebildet ist.

4. Behältnis nach einem der Ansprüche 1 bis 3, dadurch gekennzeichnet, daß der Boden (1) als polygone Fläche oder ebene Kreisfläche ausgebildet ist.

5. Behältnis nach Anspruch 4, dadurch gekennzeichnet, daß die Seitenwandung (2) im wesentlichen senkrecht vom Boden (1) abragt und daß die Trennwand (4) senkrecht zum Boden (1) angeordnet ist, wobei die Trennwand (4) vorzugsweise geradlinig durch die Mitte des Behältnisses verläuft und damit das Behältnis in zwei vorzugsweise gleich große Bereiche (3) teilt.

6. Behältnis nach Anspruch 5, dadurch gekennzeichnet, daß zur Aufteilung des Behältnisses in mehrere vorzugsweise gleich große Bereiche (3) mehrere Trennwände (4) vorgesehen sind und daß die Trennwände (4) geradlinig und sternförmig mit einem gemeinsamen Schnittpunkt in der Mitte des Behältnisses angeordnet sind.

7. Behältnis nach Anspruch 5 oder 6, dadurch gekennzeichnet, daß der Anschlag (5) stegförmig als quasi Verlängerung einer Trennwand (4) nach unten ausgebildet ist und ggf. eine geringere Länge als der Radius der Kreisfläche des Bodens (1) aufweist.

8. Behältnis nach einem der Ansprüche 1 bis 7, dadurch gekennzeichnet, daß der Boden (1), die Seitenwandung (2), die Trennwand (4) bzw. die Trennwände (4), der Anschlag (5) und ggf. das Auflageelement (6) integrale Bestandteile des Behältnisses sind.

9. Behältnis nach einem der Ansprüche 1 bis 8, dadurch gekennzeichnet, daß der Boden (1), die Seitenwandung (2), die Trennwand (4) bzw. die Trennwände (4), der Anschlag (5) und ggf. das Auflageelement (6) aus Glas hergestellt sind.

10. Behältnis nach einem der Ansprüche 1 bis 9, dadurch gekennzeichnet, daß der Boden (1), die Seitenwandung (2), die Trennwand (4) bzw. die Trennwände (4), der Anschlag (5) und ggf. das Auflageelement (6) vorzugsweise spritzgußtechnisch aus Kunststoff hergestellt sind.

11. Behältnis nach einem der Ansprüche 1 bis 10, dadurch gekennzeichnet, daß am Boden (1) den einzelnen Bereichen (3) zugeordnete, unterschiedliche Kennzeichnungen (8) vorgesehen sind, wobei die Kennzeichnungen (8) ggf. eingegossen oder eingeprägt und als alphanumerische Zeichen, arabische Ziffern oder römische Ziffern ausgeführt sind.

12. Behältnis nach einem der Ansprüche 1 bis 11, dadurch gekennzeichnet, daß die Seitenwandung (2) derart stabil ausgebildet ist, daß sie von einer Einrichtung zum Drehen des Behältnisses von oben greifbar ist.

13. Behältnis nach einem der Ansprüche 1 bis 12, dadurch gekennzeichnet, daß ein Deckel zum Verschließen des Behältnisses vorgesehen ist, der ggf. die Seitenwandung (2) übergreift.

## Claims

1. Container for receiving solid and/or fluid media, in particular nutrient solutions or nutrient media for bacterial cultures, having a base (1), a side wall (2), which extends from the base (1), and at least one dividing wall (4), which subdivides the container into separate regions (3), characterised in that, at a predetermined point of the container, a stop (5) is arranged for the angular positioning of the container relative to an axis of rotation which extends substantially vertically through the base (1).

2. Container according to claim 1, characterised in that the stop (5) is arranged on the underside of the base (1) and extends from the underside of the base (1).

3. Container according to claim 2, characterised in that the base (1) has a downwardly projecting support element (6) for support on a support surface, in that the support element (6) extends from the base (1) at least as far as the stop (5) and, optionally, is in the form of a base rim (7) which extends at least partially around the circumference of the base.

4. Container according to any one of claims 1 to 3, characterised in that the base (1) is in the form of a polygon face or a regular circular face.

5. Container according to claim 4, characterised in that the side wall (2) extends substantially perpendicularly from the base (1), and in that the dividing wall (4) is arranged perpendicularly relative to the base (1), the dividing wall (4) extending preferably in a rectilinear manner through the centre of the container and, therefore, dividing the container into two regions (3) preferably of identical size.

6. Container according to claim 5, characterised in that several dividing walls (4) are provided to subdivide the container into several regions (3) which are preferably of identical size, and in that the dividing walls (4) are arranged in a rectilinear manner and in the shape of a star having a common point of intersection at the centre of the container.

7. Container according to claim 5 or 6, characterised in that the stop (5) is fin-shaped and in the form of a type of downward extension of a dividing wall (4) and optionally has a shorter length than the radius of the circular face of the base (1).

8. Container according to any one of claims 1 to 7, characterised in that the base (1), the side wall (2), the dividing wall(s) (4), the stop (5) and, optionally, the support element (6) are integral components of the container.

9. Container according to any one of claims 1 to 8, characterised in that the base (1), the side wall (2), the dividing wall(s) (4), the stop (5) and, optionally, the support element (6) are of glass.

10. Container according to any one of claims 1 to 9, characterised in that the base (1), the side wall (2), the dividing wall(s) (4), the stop (5) and, optionally, the support element (6) are preferably produced from a plastics material using an injection moulding technique.

11. Container according to any one of claims 1 to 10, characterised in that there are provided on the base (1) various designations (8) which are associated with the individual regions (3), the designations (8) optionally being cast or stamped and being in the form of alpha-numerical characters, Arabic numerals or Roman numerals.

12. Container according to any one of claims 1 to 11, characterised in that the side wall (2) is of such stable form that it can be gripped from above by a device for rotating the container.

13. Container according to any one of claims 1 to 12, characterised in that a lid is provided for closing the container and optionally engages over the side wall (2).

## Revendications

1. Récipient destiné à recevoir des solides et/ou des liquides, en particulier des solutions ou substances nutritives pour des cultures bactériologiques, avec un fond (1), une paroi latérale (2) issue du fond (1), et au moins une paroi de séparation (4) compartimentant le récipient en secteurs (3) distincts
caractérisé
en ce qu'est disposé à un endroit prédéterminé du récipient une butée (5) destinée au positionnement angulaire du récipient par rapport à un axe de rotation qui s'étend au travers du fond, perpendiculairement à lui.

2. Récipient suivant la revendication 1
caractérisé en ce que la butée (5) est disposée sur la face intérieure du fond (1) et dépasse de la face intérieure du fond (1).

3. Récipient suivant la revendication 2
caractérisé
en ce que le tond (1) présente un élément de support (6) s'étendant vers le bas pour la pose sur une base,
en ce que l'élément de support (6) dépasse du fond (1) au moins autant que la butée (5), et adopte, le cas échéant, la forme d'un bord (7) du tond qui s'étend au moins en partie le long de la périphérie du fond.

4. Récipient suivant l'une quelconque des revendications 1 à 3
caractérisé
en ce que le fond (1) a la forme d'une surface polygonale ou d'une surface circulaire plane.

5. Récipient suivant la revendication 4
caractérisé
en ce que la paroi latérale (2) s'élève en substance à la verticale à partir du tond (1) et que la paroi de séparation (4) est disposée perpendiculairement par rapport au fond (1), la paroi de séparation (4) passant de préférence, en ligne droite, par le centre du récipient et divisant dès lors le récipient en deux secteurs (3) qui sont de préférence de la même étendue.

6. Récipient suivant la revendication 5
caractérisé
en ce que plusieurs parois de séparation (4) sont prévues pour la division du récipient en plusieurs secteurs (3) qui sont de préférence d'égale étendue et
en ce que les parois de séparation (4) sont disposées en étoile, les branches de l'étoile passant, en ligne droite, par un point d'intersection commun au centre du récipient.

7. Récipient suivant la revendication 5 ou la revendication 6
caractérisé
en ce que la butée (5) a la forme d'une nervure se trouvant quasiment dans le prolongement vers le bas d'une paroi de séparation (4) et présente, le cas échéant, une longueur inférieure au rayon de la surface circulaire du fond (1).

8. Récipient suivant l'une quelconque des revendications 1 à 7
caractérisé
en ce que le fond (1), la paroi latérale (2), la ou les paroi(s) de séparation (4), la butée (5) et, le cas échéant, l'élément de support (6), font partie intégrante du récipient.

9. Récipient suivant l'une quelconque des revendications 1 à 8
caractérisé
en ce que le fond (1), la paroi latérale (2), la ou les paroi(s) de séparation (4), la butée (5) et, le cas échéant, l'élément de support(6), sont réalisés en verre.

10. Récipient suivant l'une quelconque des revendications 1 à 9
caractérisé
en ce que le fond (1), la paroi latérale (2), la ou les paroi(s) de séparation (4), la butée (5) et, le cas échéant, l'élément de support(6), sont réalisés de préférence par la technique du moulage par injection de matière plastique.

11. Récipient suivant l'une quelconque des revendications 1 à 10
caractérisé
en ce que en ce que sont prévues sur le fond (1) des marques (8) distinctes assignées aux différents secteurs (3), ces marques (8) pouvant être moulées ou imprimées dans la masse et se présenter sous forme de signes alphanumériques, de chiffres arabes ou romains.

12. Récipient suivant l'une quelconque des revendications 1 à 11
caractérisé
en ce que la paroi latérale (2) est réalisée assez solidement pour pouvoir être saisie du haut par un dispositif destiné à faire tourner le récipient.

13. Récipient suivant l'une quelconque des revendications 1 à 12
caractérisé
en ce qu'est prévu un couvercle de fermeture du récipient qui peut éventuellement chevaucher la paroi latérale (2).
